# EUROPEAN PATENT APPLICATION

(11) **EP 3 095 428 A1**
(43) Date of publication of application: **23.11.2016**
(21) Application number: 14878763.3
(22) Date of filing: 22.10.2014
(51) Int. Cl.: A61G 15/08, A47C 3/20, A61F 2/68, A61H 3/00, B25J 11/00

(54) **WORKING POSTURE HOLDING DEVICE**

(30) Priority: 15.01.2014 JP 2014005179
(71) Applicant: Panasonic Corporation, Kadoma-shi, Osaka 571-8501 (JP)
(72) Inventor: NAKATANI, Kazuhiro, Osaka-shi, Osaka 540-6207 (JP); KONISHI, Makoto, Osaka-shi, Osaka 540-6207 (JP); FUJIMOTO, Hiromichi, Osaka-shi, Osaka 540-6207 (JP)
(74) Representative: Eisenführ Speiser
(86) International application number: PCT/JP2014/005366
(87) International publication number: WO 2015/107576

(57) **Abstract**

A working posture holding device (10) includes: a lower back holding part (11) that holds a lower back of a user; a leg part (12a) that supports the lower back holding part, the leg part being rotatably connected to the lower back holding part, the leg part extending along a leg region of the user; a foot part (13a) that is connected to the leg part, the foot part contacting a ground to support the overall working posture holding device; a fixing part (14a) that fixes the leg part and the user to each other; and a locking mechanism (15a) that switches a state of the leg part between a "variable" state where a distance between a connection part to the lower back holding part and a connection part to the foot part is variable, and a "locked" state where the distance is fixed, wherein when the locking mechanism sets the leg part to be in the locked state, the lower back holding part holds a lower back of the user to hold a standing posture of the user and, when the locking mechanism sets the leg part to in the variable state, the user is able to walk.

## Description

### TECHNICAL FIELD

This disclosure relates to a working posture holding device to be worn on a body of a user to hold his/her standing posture that continues for a long time.

### BACKGROUND ART

A surgeon is traditionally forced to maintain his/her standing posture during a surgical operation that continues for a long time. In this case, though a simple chair may be used, such a chair does not sufficiently remove the physical burden of the surgeon because of the restriction on the location to place the chair and of the case where the surgeon needs to move during a surgical operation.

A chair to be used during a surgical operation is known whose height is adjustable as shown in Japanese Patent Laid-Open Publication No. 52-44055 referred as Patent Document 1 while this chair can provide its adjustable height for a surgeon only in his/her sitting posture. This chair is therefore not different from any traditional chair in that the surgeon needs to leave the chair to work when the surgeon moves during, before, and after a surgical operation.

### SUMMARY

An object of this disclosure is to provide a working posture holding device that holds a standing posture of a person continuing for a long time and that does not obstruct any move of the person.

A working posture holding device comprising:
a lower back holding part that holds a lower back of a user;
a leg part that supports the lower back holding part, the leg part being rotatably connected to the lower back holding part, the leg part extending along a leg region of the user;
a foot part that is connected to the leg part, the foot part contacting a ground to support the overall working posture holding device;
a fixing part that fixes the leg part and the user to each other; and
a locking mechanism that switches a state of the leg part between a "variable" state where a distance between a connection part to the lower back holding part and a connection part to the foot part is variable, and a "locked" state where the distance is fixed, wherein
when the locking mechanism sets the leg part to be in the locked state, the lower back holding part holds a lower back of the user to hold a standing posture of the user and, when the locking mechanism sets the leg part to in the variable state, the user is able to walk.

According to the working posture holding device of this disclosure, when the locking mechanism sets the leg part to be in the "locked" state, the lower back holding part holds the lower back of the user and thereby holds the standing posture of the user that continues for a long time and, when the locking mechanism sets the leg part to be in the "variable" state, the user can walk.

### BRIEF DESCRIPTION OF DRAWINGS

Fig. 1 is a block diagram of a configuration of a working posture holding device according to a first embodiment.
Fig. 2 is a perspective view of an overview of the working posture holding device according to the first embodiment.
Fig. 3 is a front view of the working posture holding device of Fig. 2.
Fig. 4 is a side view of the working posture holding device of Fig. 2.
Fig. 5 is a schematic view of an example where a user wears the working posture holding device of Fig. 2.
Fig. 6 is a perspective view of an overview of a working posture holding device according to a second embodiment.
Fig. 7 is a schematic view of a configuration of a leg part of a working posture holding device according to a third embodiment.

### DETAILED DESCRIPTION

As a working posture holding device of a first aspect, a working posture holding device includes:
a lower back holding part that holds a lower back of a user;
a leg part that supports the lower back holding part, the leg part being rotatably connected to the lower back holding part, the leg part extending along a leg region of the user;
a foot part that is connected to the leg part, the foot part contacting a ground to support the overall working posture holding device;
a fixing part that fixes the leg part and the user to each other; and
a locking mechanism that switches a state of the leg part between a "variable" state where a distance between a connection part to the lower back holding part and a connection part to the foot part is variable, and a "locked" state where the distance is fixed, wherein
when the locking mechanism sets the leg part to be in the locked state, the lower back holding part holds a lower back of the user to hold a standing posture of the user and, when the locking mechanism sets the leg part to in the variable state, the user is able to walk.

As a working posture holding device of a second aspect, in the first aspect, the leg part may include a lower leg part on a side of the foot part, an upper leg part on a side of the lower back holding part, and a knee part that connects the lower leg part and the upper leg part as being rotatably against each other.

As a working posture holding device of a third aspect, in the second aspect, the locking mechanism may lock rotations of the lower leg part and the upper leg part at the knee part to fix the distance between the connection part to the lower back holding part and the connection part to the foot part.

As a working posture holding device of a fourth aspect, in the first aspect, the leg part may be stretchable for the distance between the lower back holding part and the foot part to be variable, and
the locking mechanism may lock stretching and shortening of the leg part to fix the distance between the connection part to the lower back holding part and the connection part to the foot part.

As a working posture holding device of a fifth aspect, in any one of the first to fourth aspects, the locking mechanism may lock a rotation of the leg part at the connection part of the leg part to the lower back holding part.

As a working posture holding device of a sixth aspect, in in any one of the first to fifth aspects, the locking mechanism may further lock the connection part of the leg part to the foot part.

As a working posture holding device of a seventh aspect, in in any one of the first to sixth aspects, the working posture holding device may further include a motor disposed in the connection part of the leg part to the lower back holding part to enable the leg part to rotate.

As a working posture holding device of a eighth aspect, in the seventh aspect, the working posture holding device may further include a balance control part that executes balance control using the motor disposed in the connection part between the leg part and the lower back holding part.

As a working posture holding device of a ninth aspect, in in any one of the first to sixth aspects, the lower back holding part may include a saddle that supports buttocks of the user.

The working posture holding devices according to embodiments of this disclosure will be described with reference to the accompanying drawings. In the drawings, the substantially same members are given the same reference numerals.

### (First Embodiment)

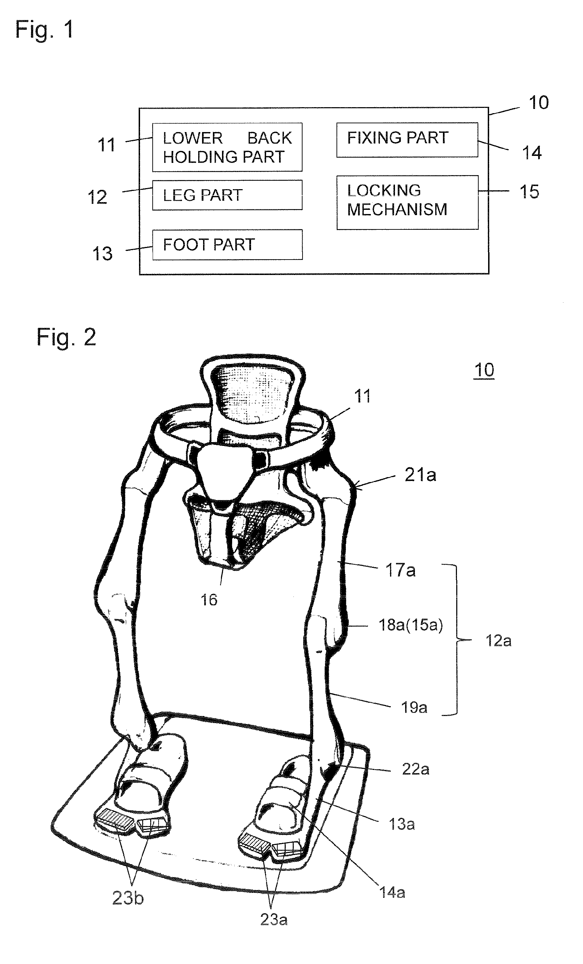

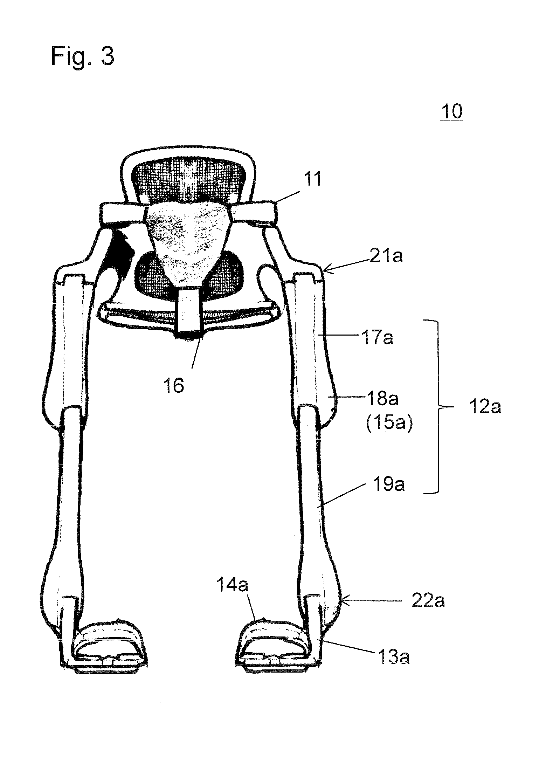

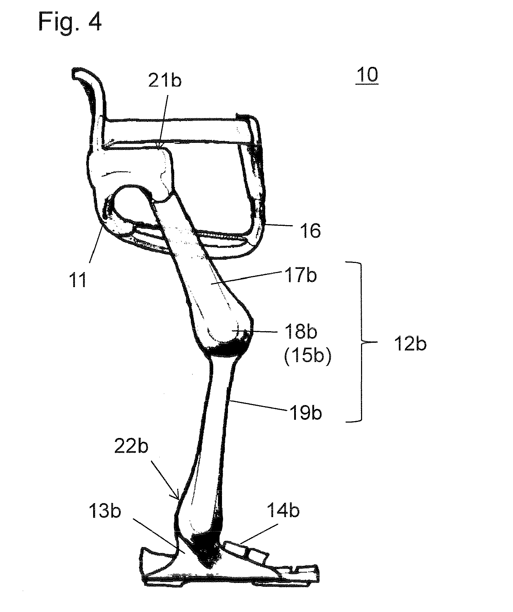

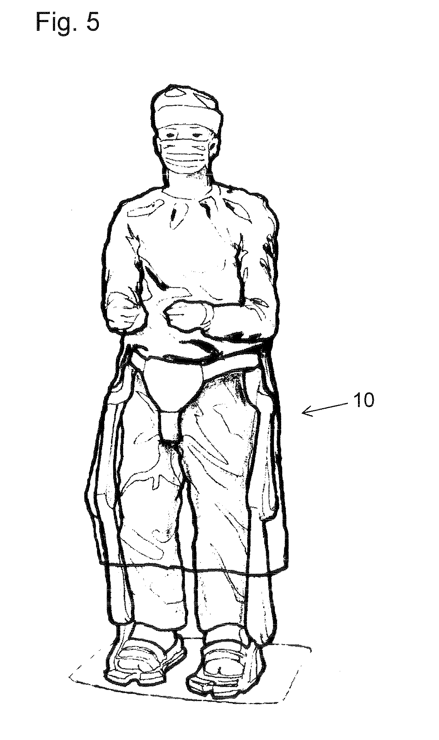

Fig. 1 is a block diagram of a configuration of the working posture holding device 10 according to the first embodiment. Fig. 2 is a perspective view of an overview of the working posture holding device 10 according to the first embodiment. Fig. 3 is a front view of the working posture holding device 10 of Fig. 2. Fig. 4 is a side view of the working posture holding device 10 of Fig. 2. Fig. 5 is a schematic view of an example where a user wears the working posture holding device 10 of Fig. 2.

The working posture holding device 10 according to the first embodiment includes a lower back holding part 11 that holds the lower back of a user, leg parts 12, 12a, and 12b that support the lower back holding part 11, foot parts 13, 13a, and 13b that are connected to the leg parts 12, 12a, and 12b, and that contact the ground to support the overall apparatus, fixing parts 14, 14a, and 14b that fix the leg parts 12, 12a, 12b, and the user to each other, and locking mechanisms 15, 15a, and 15b. The leg parts 12, 12a, and 12b are further rotatably connected to the lower back holding part 11 to extend along the leg regions of the user. The locking mechanisms 15, 15a, and 15b switch a state of the leg parts 12, 12a, and 12b between a "variable" state where the distance is variable between a connection part thereof to the lower back holding part 11 and a connection part thereof to each of the foot parts 13, 13a, and 13b, and a "locked" state where the distance is fixed.

The lower back holding part 11 thereby holds the lower back of the user to hold his/her standing posture when the locking mechanisms 15 and 15a set the leg parts 12 and 12a to be in the locked state, and the user can walk when the locking mechanisms 15 and 15a set the leg parts 12 and 12a to be in the variable state. This is because, when the locking mechanisms 15 and 15a set the leg parts 12 and 12a to be in the locked state, for the standing posture of the user, the distance is fixed between the connection part to the lower back holding part 11 and the connection parts to the foot parts 13, 13a, and 13b, and the user can therefore maintain the angle formed by the femoral region and the lower leg region sandwiching the knee region without applying any significant load thereto. The user can therefore maintain his/her standing posture with a reduced burden on his/her knee regions.

The constituent members constituting the working posture holding device will be described.

### <Lower Back Holding Part>

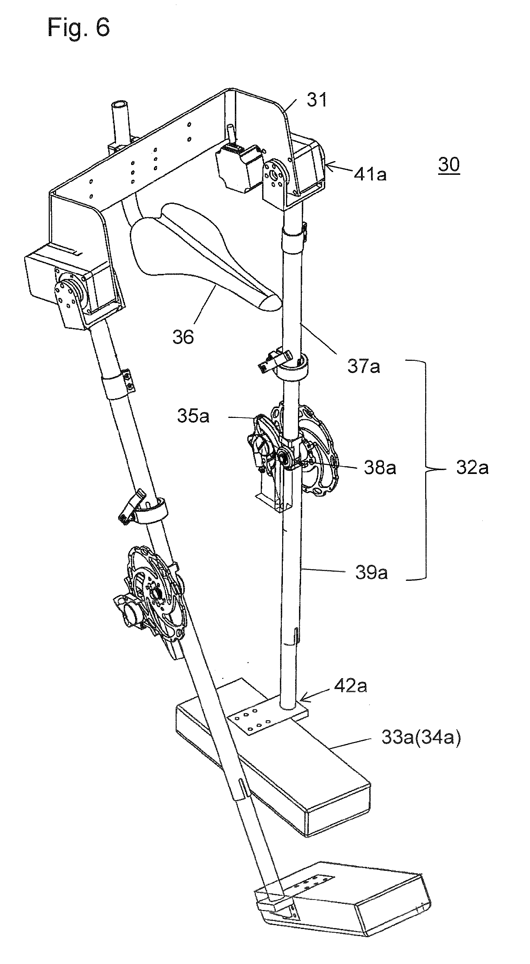

The lower back holding part 11 is disposed in the vicinity (an area) of the lower back of the user to hold the lower back. For example, the lower back holding part 11 includes a saddle 16 that supports the buttocks of the user and a housing 17 that supports the saddle. The saddle 16 includes a net-like seating face, supports the buttocks of the user with a large area, and thereby can reduce the burden of the user. The lower back holding part 11 rotatably supports the left and the right leg parts 12a and 12b independently from each other in the housing 17. The saddle 16 is not limited to the one depicted in Fig. 2 that includes the net-like seating face and, for example, a saddle may be the one that includes a seating face of a bicycle saddle, or the like such as, for example, a saddle 36 of a working posture holding device according to the second embodiment depicted in Fig. 6. Otherwise, the lower back holding part 11 may be a harness-type lower back holding part that supports the lower back of the user using a harness instead of the saddle.

### <Leg Part>

The left and the right leg parts 12a and 12b are respectively connected to the foot parts 13a and 13b at ends thereof, and the lower back holding part 11 at the other ends thereof. The leg parts 12a and 12b extend along the legs of the user. For example, the leg parts 12a and 12b extend along the lateral sides of the legs of the user. The leg parts 12a and 12b may extend along the front sides or back sides of the legs of the user. When the leg parts 12, 12a, and 12b extend on the lateral sides of the legs of the user, this is advantageous because the user can work without being conscious of the presence of the leg parts 12, 12a, and 12b. When the leg parts 12, 12a, and 12b extend on the front sides or the back sides of the legs of the user, this is advantageous because the user can easily execute the attachment work of the leg parts 12, 12a, and 12b.

The leg parts 12a and 12b may further include upper leg parts 17a and 17b on the side of the lower back holding part 11, lower leg parts 19a and 19b on the side of the foot parts 13a and 13b, and knee parts 18a and 18b that respectively connect the upper leg parts 17a and 17b and the lower leg parts 19a and 19b as being rotatably against each other. The knee parts 18a and 18b may each include a backstop mechanism (not depicted) that deters any frontward rotation of the lower leg parts 19a and 19b that center the knee parts 18. The working posture holding device 10 can thereby avoid any reverse rotation state of the knees that cannot be realized by each of the knee regions of the user, and can safely be used.

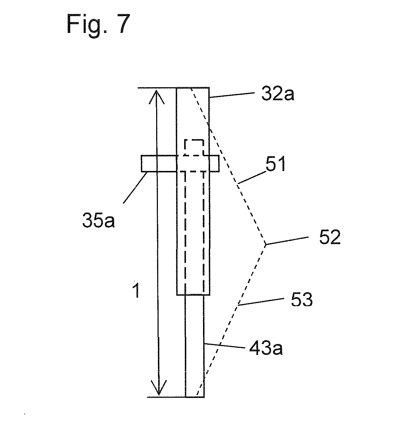

As depicted in Fig. 7, the distance l between the lower back holding part 11 and the foot part 33a may be set to be variable by setting a leg part 32a to be stretchable without disposing any knee part in the leg part 32a.

In the leg parts 12a and 12b, a motor may be disposed in each of the connection parts (lower back joint parts 21 a and 41 a) to enable the leg parts 12a and 12b to rotate. A motor may also be disposed in each of the knee parts 18a and 18b to enable the upper leg parts 17a and 17b and the lower leg parts 19a and 19b to rotate against each other. A motor may also be disposed in each of the connection parts (ankle joint parts 22a and 22b) to the foot parts 13a and 13b.

Balance control parts (not depicted) may further be disposed, that execute balance control using motors (not depicted) disposed in the connection pars (the lower back joint parts 21 a and 41 a) between the leg parts 12a and 12b, and the lower back holding part 11. The balance control can suppress any back-and-forth waving and the user can lean on the apparatus like an ordinary chair.

As depicted in Fig. 2 to Fig. 5, the knee parts 18a and 18b may each be disposed in the vicinity (an area) of the knee region of the user. Otherwise, the knee parts may each be disposed not in front of but in back of the knee regions of the user like a bird foot (not depicted).

### <Foot Part>

The foot parts 13a and 13b are connected to the leg parts 12a and 12b, and contact the ground to support the overall apparatus. In Fig. 2 to Fig. 5, the foot parts 13a and 13b are disposed being integrated with sandal parts of the user. The foot parts 13a and 13b are not limited by the above example and, for example, may be those only to place the feet of the user thereon like the ones depicted as the foot parts 33a of the work posture holding device described in the second embodiment. Otherwise, the foot parts 13a and 13b may be those that contact the ground to hold the overall apparatus irrespective of the feet of the user. In this case, the foot parts 13a and 13b may be disposed on the outer side of the feet of the user.

### <Fixing Part>

The fixing parts 14a and 14b fix the leg parts 12, 12a, and 12b and the user to each other. The fixing parts 14a and 14b may be realized by, for example, the sandal parts of the foot parts 13a and 13b. Otherwise, the fixing parts 14a and 14b may be those that fix the intermediate portions of the leg parts 12a and 12b, and the leg regions of the user to each other using bands, or the like.

### <Locking Mechanism>

The locking mechanisms 15a and 15b switch a state of the leg parts 12, 12a, and 12b between the variable state where the distance between the connection parts to the lower back holding part 11 and the connection parts to the foot parts 13, 13a, and 13b is variable, and the locked state where the distance is fixed. The locking mechanisms 15a and 15b, for example, may lock the rotations of the lower leg parts 19a and 19b, and the upper leg parts 17a and 17b against each other at the knee parts 18a and 18b to fix the distance between the connection parts to the lower back holding part 11 and the connection parts to the foot parts 13a and 13b. When the leg part 32a is stretchable and the distance l between the lower back holding part 11 and the foot part 33a is thereby variable like a locking mechanism 35a (Fig. 7) according to a third embodiment described later, the locking mechanism 35a may lock the stretching and the shortening of the leg part 32a.

Thereby, when the locking mechanisms 15 and 15a set the leg parts 12 and 12a to be in the locked state, the lower back holding part 11 holds the lower back of the user to hold his/her standing posture and, when the locking mechanisms 15 and 15a set the leg parts 12 and 12a to be in the variable state, the user can walk.

The locking mechanisms may lock the rotations of the leg parts 12a and 12b at the connection parts (the lower back joint parts 21a and 41a) of the leg parts 12a and 12b to the lower back holding part 11. The locking mechanisms may further fix the connection parts (the ankle joint parts 22a and 42a) of the leg parts 12a and 12b to the foot parts 13a and 13b.

The standing posture can thereby be more stably held by executing not only the fixation by the knee parts 18a and 18b but also the fixation by the lower back holding part 11 and further the fixation by foot parts 13a and 13b.

Each of the locking mechanisms 15a and 15b can employ any usable locking scheme such as, for example, a mechanical lock such as the one of a disc brake type or a gear type, or an electromagnetic lock. For example, a lever, a switch, a sensor, or the like may be used to turn on and turn off each of the locking mechanisms 15a and 15b. For example, in the example of Fig. 2, the locking mechanisms 15a and 15b may be turned on or turned off using foot switches 23a and 23b each capable of being operated by toes.

When the locked state set by the locking mechanisms 15a and 15b is cancelled, the variable state is established where the distance is variable between the connection parts to the lower back holding part 11 and the connection parts to the foot parts 13, 13a, and 13b. The user can therefore freely walk because the fixation of each of his/her knee regions is cancelled. In this case, during the walking, the working posture holding device 10 moves along with the feet of the user and does therefore not significantly bother the user.

### (Second Embodiment)

Fig. 6 is a perspective view of an overview of the working posture holding device 30 according to the second embodiment.

The working posture holding device 30 according to the second embodiment differs from the working posture holding device 10 according to the first embodiment in a comparison therebetween in that a saddle used in, for example, a bicycle is used as the saddle 36 and that a disc brake type locking mechanism 35a is disposed in a knee part 38a.

The working posture holding device 30 can easily be manufactured by using the saddle used in a bicycle or the like as the saddle 36 as above. The working posture holding device 30 can easily be manufactured and the knee part 38a can reliably be locked by using the disc brake type locking mechanism 35a.

### (Third Embodiment)

Fig. 7 is a schematic view of a configuration of the leg part 32a of the working posture holding device according to the third embodiment.

The working posture holding device according to the third embodiment has the substantially same configuration as that of the working posture holding device 30 according to the second embodiment except the configuration of the leg part 32a. The leg part 32a of the working posture holding device according to the third embodiment differs from the leg part 32a of the working posture holding device 30 according to the second embodiment in a comparison therebetween in that no knee part is disposed in the leg part 32a for the leg part 32a to be stretchable to enable the variation of the distance l between the lower back holding part 11 and the foot part 33a. For example, the distance l is varied by the stretching or the shortening of the stretchable part 43a from the leg part 32a. In this case, as depicted in Fig. 7, because the leg part 32a includes no knee part, the femoral region 51, the knee region 52, and the lower leg region 53 of the user do not correspond to any part of the leg part 32a. On the other hand, because the leg part 32a is stretchable, the leg part 32a can correspond to any variation of the distance l between the lower back and the ankle.

The working posture holding device according to the third embodiment includes the locking mechanism 35a that locks the stretching and the shortening of the leg part 32a. This locking mechanism 35a can deter the stretching and the shortening of the stretchable part 43a from the leg part 32a and can fix the distance between the connection part to the lower back holding part and the connection part to the foot part. The lower back holding part can thereby hold the lower back of the user and can hold his/her standing posture that continues for a long time.

According to the working posture holding device of this disclosure, when the locking mechanism sets the leg part to be in the locked state, the lower back holding part can hold the lower back of the user and can hold his/her standing posture that continues for a long time and, when the locking mechanism sets the leg part to be in the variable state, the user can walk. The working posture holding device is useful for uses of the working posture holding device to support any work done in a standing posture for a long time by, such as a surgeon, a pastor, or the like, work done in a standing posture in a kitchen, a cooking area, or the like by a homemaker, a cook, or the like.

### EXPLANATIONS OF LETTERS OR NUMBERS

- 10, 30: working posture holding device
- 11, 31: lower back holding part
- 12, 12a, 32a: leg part
- 13, 13a, 33a: foot part
- 14, 14a, 34a: fixing part
- 15, 15a, 35a: locking mechanism
- 16, 36,: saddle (of a net type)
- 17a, 17b, 37a: upper leg part
- 18a, 18b, 38a: knee part
- 19a, 19b, 39a: lower leg part
- 21a, 41a: lower back joint part
- 22a, 42a: ankle joint part
- 23a, 23b: foot switch
- 43a: stretchable part
- 51: femoral region (user)
- 52: knee region (user)
- 53: lower leg region (user)

## Claims

1. A working posture holding device comprising:
a lower back holding part that holds a lower back of a user;
a leg part that supports the lower back holding part, the leg part being rotatably connected to the lower back holding part, the leg part extending along a leg region of the user;
a foot part that is connected to the leg part, the foot part contacting a ground to support the overall working posture holding device;
a fixing part that fixes the leg part and the user to each other; and
a locking mechanism that switches a state of the leg part between a "variable" state where a distance between a connection part to the lower back holding part and a connection part to the foot part is variable, and a "locked" state where the distance is fixed, wherein
when the locking mechanism sets the leg part to be in the locked state, the lower back holding part holds a lower back of the user to hold a standing posture of the user and, when the locking mechanism sets the leg part to in the variable state, the user is able to walk.

2. The working posture holding device according to claim 1, wherein
the leg part comprises a lower leg part on a side of the foot part, an upper leg part on a side of the lower back holding part, and a knee part that connects the lower leg part and the upper leg part as being rotatably against each other.

3. The working posture holding device according to claim 2, wherein
the locking mechanism locks rotations of the lower leg part and the upper leg part at the knee part to fix the distance between the connection part to the lower back holding part and the connection part to the foot part.

4. The working posture holding device according to claim 1, wherein
the leg part is stretchable for the distance between the lower back holding part and the foot part to be variable, wherein
the locking mechanism locks stretching and shortening of the leg part to fix the distance between the connection part to the lower back holding part and the connection part to the foot part.

5. The working posture holding device according to any one of claims 1 to 4, wherein
the locking mechanism locks a rotation of the leg part at the connection part of the leg part to the lower back holding part.

6. The working posture holding device according to any one of claims 1 to 5, wherein
the locking mechanism further locks the connection part of the leg part to the foot part.

7. The working posture holding device according to any one of claims 1 to 6, further comprising
a motor disposed in the connection part of the leg part to the lower back holding part to enable the leg part to rotate.

8. The working posture holding device according to claim 7, further comprising
a balance control part that executes balance control using the motor disposed in the connection part between the leg part and the lower back holding part.

9. The working posture holding device according to any one of claims 1 to 8, wherein
the lower back holding part comprises a saddle that supports buttocks of the user.
